(19) Europäisches Patentamt
European Patent Office
Office européen des brevets

(11) EP 1 066 336 B1

(12) # EUROPÄISCHE PATENTSCHRIFT

(45) Veröffentlichungstag und Bekanntmachung des Hinweises auf die Patenterteilung:
**25.05.2005 Patentblatt 2005/21**

(21) Anmeldenummer: **99919163.8**

(22) Anmeldetag: **30.03.1999**

(51) Int Cl.[7]: **C08K 3/00**, C08K 5/00, A61K 6/10

(86) Internationale Anmeldenummer:
**PCT/EP1999/002192**

(87) Internationale Veröffentlichungsnummer:
**WO 1999/050344 (07.10.1999 Gazette 1999/40)**

(54) **SELBSTDESINFIZIERENDE KUNSTSTOFFE UND IHRE VERWENDUNG IM DENTALEN UND DENTALTECHNISCHEN BEREICH**

SELF-DESINFECTING PLASTICS AND THE USE THEREOF IN DENTISTRY AND DENTAL TECHNOLOGY

MATIERES PLASTIQUES AUTO-DESINFECTANTES ET LEUR UTILISATION DANS LE DOMAINE DENTAIRE ET DE TECHNIQUE DENTAIRE

(84) Benannte Vertragsstaaten:
**DE FR GB IT NL**

(30) Priorität: **30.03.1998 DE 19814133**

(43) Veröffentlichungstag der Anmeldung:
**10.01.2001 Patentblatt 2001/02**

(73) Patentinhaber: **3M ESPE AG**
**82229 Seefeld (DE)**

(72) Erfinder: **GANGNUS, Bernd**
**D-82346 Andechs (DE)**

(56) Entgegenhaltungen:
**EP-A- 0 360 962**
**EP-A- 0 606 762**
**WO-A-93/21970**
**WO-A-94/14897**
**GB-A- 2 210 889**
**US-A- 4 647 601**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 1 066 336 B1

**Beschreibung**

Die Erfindung betrifft Kunststoffe, die selbstdesinfizierend sind. Im besonderen betrifft die Erfindung dentale Kunststoffe und Abformmassen, in denen ein biozides Material inkorporiert und deren Oberfläche gegenüber Mikroorganismen adhäsionsverringert ist.

Desinfektion ist ein permanentes Bedürfnis in Zahnarztpraxen und zahntechnischen Labors. Die Übertragung von Keimen von kontaminierten Handschuhen oder sogar bloßen Händen über Gerätschaften, Werkzeuge oder Materialien auf den Menschen stellt ein enormes Risiko für den Patienten, den Zahnarzt oder -techniker und deren Assistenten dar. Besonders im täglichen Arbeitsablauf in der Praxis kann der Patient mit kontaminierten Gegenständen oder Massen in Berührung kommen, wobei durch offene Wunden Mikroorganismen in die Blutbahn eindringen können. In einer Sitzung kann es beispielsweise durch Verwendung kontaminierter Handschuhe beim Mischen einer Abformmasse und bei der Verwendung kontaminierter Lichtleiter zur Lichtpolymerisation von strahlungshärtbaren Füllungsmaterialien zur Erhöhung des Infektionsrisikos kommen. Zwar sind die Hygienevorschriften sehr streng, doch kann kein absolut zuverlässiger Schutz vor Infektion gewährleistet werden.

Die Verwendung biozider Substanzen ist auf anderen Gebieten, beispielsweise zur Herstellung von plastifiziertem Polyvinylchlorid zur Verwendung als Swimming Pool-Reiniger, bekannt (C.R. Jones, P.S. Handley, G.D. Robson, I.M. Eastwood and M. Greenhalgh "Biocides incorporated into plasticized polyvinylchlorid reduce adhesion of Pseudomonas fluorescens BL146 and substratum hydrophobicity", Journal of Applied Bacteriology (1996) 81, 553-560). Nachteilig ist hierbei, daß die biozide Wirkung der in den Kunststoff eingebrachten Wirkstoffe 10,10-Oxybisphenoxyarsin (OBPA), 2-n-Octyl-4-isothiazolin-3-on (OIT), 2,3,5,6-Tetrachloro-4-(methylsulphonyl)pyridin (TCMP) und N-Trichloromethylthiophthalimid (NCMP) nur dann voll zur Geltung kommt, wenn die abzutötenden Mikroorganismen direkt in Kontakt mit der Oberfläche kommen. Die dazu notwendige Erniedrigung der Adhäsion wird zufällig durch die verwendeten bioziden Substanzen selbst erzielt.

Biozide Wirkung entfalten aber auch Silber, Kupfer oder Zink enthaltende Substanzen, beispielsweise Produkte der Firma DuPont (MicroFree™ AMP). Eingebettet in die verschiedensten Gegenstände aus Kunststoff wird eine antimikrobielle Wirkung auf den Oberflächen solcher modifizierter Kunststoffe verzeichnet.

Ein anderer Ansatz ist die Verringerung der Adhäsion von Proteinen auf Oberflächen. Da Zellmembranen von Mikroorganismen gleichfalls aus Proteinbestandteilen aufgebaut sind, versucht man, durch die Adhäsionsverringerung von Proteinen die Anlagerung von Mikroorganismen aber besonders auch von Blutbestandteilen zu verhindern. Ein umfangreicher Stand der Technik beschreibt Methoden zur Veränderung der Eigenschaften von Kunststoffoberflächen oder ganzen Kunststoffteilen, um diese biokompatibel zu machen. Katheter, Prothesen und Implantate erhalten durch die beschriebenen Methoden biokompatible Oberflächen. Die Ablagerung von biologischem Material, beispielsweise Blutzellen auf Prothesen oder Kathetern oder die Ablagerung von Proteinen aus der Tränenflüssigkeit auf Kontaktlinsen kann verringert werden, wenn spezielle Kunststoffe zur Herstellung solcher Gegenstände verwendet oder bereits vorgeformte Gegenstände mit speziellen Polymeren beschichtet werden.

Die WO-93/01221 beschreibt beispielsweise Polymere aus einem oder mehreren radikalisch polymerisierbaren Monomeren, wobei diese Polymere Gruppen mit permanenter positiver Ladung und Gruppen, die zur Ausbildung einer Bindung an Oberflächen befähigt sind, besitzen.

Ein weiterer Ansatz ist aus der WO-94/14897 bekannt, worin die Biokompatibilität von Polymeren durch die Verknüpfung mit zwitterionische Gruppen enthaltenden Polymeren erhalten wird. Die zwitterionische Gruppe ist üblicherweise ein Ammoniumsalz-Ester; das Polymer, welches diese Gruppe enthält, kann über die freie radikalische Polymerisation von ethylenisch ungesättigten Monomeren zusammen mit einem Monomer, welches eine zwitterionische Gruppe enthält, hergestellt werden.

Verbindungen, die Phosphorylcholingruppen enthalten, sind bekannt für ihre hämokompatibilisierenden Eigenschaften und für die adhäsionsverringernde Wirkung von Blutplättchen auf Oberflächen, die mit diesen Verbindungen behandelt oder aus solchen Verbindungen hergestellt wurden. WO-93/21970 beschreibt beispielsweise Methoden zur Herstellung von Oberflächen, die Gruppen der allgemeinen Formel

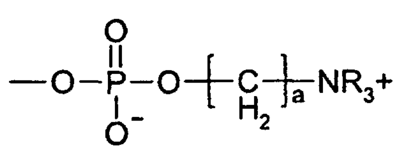

enthalten, worin R gleich oder verschieden sein kann und einen Alkylrest mit 1 bis 4 C-Atomen darstellt und a von 1 bis 4 reichen kann.

M. Humphries, J.F. Jaworzyn, J.B. Cantwell und A. Eakin beschrieben in FEMS Microbiology Letters (1987)

42, 91-101 die Benutzung von nicht-ionischen ethoxylierten und propoxylierten Surfaktanten, um die Adhäsion von Bakterien an festen Oberflächen zu verringern. Im besonderen werden dort alkoholische Ethoxylate und Alkylphenolethoxylate (ICI Plastics and Petrochemicals Division, Wilton, U.K.) verwendet. In FEMS Microbiology Ecology (1986) 38, 299-308 beschreiben M. Humphries, J.F. Jaworzyn und J.B. Cantwell die Verwendung biologischer Polymere und synthetischer Surfaktanten, um die Adhäsion gewisser Bakterienspezies durch die Erzeugung hydrophober Oberflächen zu verringern.

Es ist allerdings bekannt, daß es nicht genügt, die Oberflächen entweder hydrophob oder hydrophil zu gestalten, um die Adhäsion von Mikroorganismen zu erniedrigen, da die Beschaffenheit der Zellwände je nach Spezies vollkommen unterschiedlich sein kann.

In die Reihe der Beschichtungsverfahren gliedert sich auch die Methode von A. Pavesio, D. Renier, C. Cassinelli und M. Morra in Medical Device Technology (September 1997), 20ff ein, worin antiadhäsive Oberflächen durch Beschichtungen mit Hyaluronderivaten beschrieben werden.

Neuere Beschichtungsmittel, beinhaltend Polymere mit Wiederholungseinheiten mit polaren oder polarisierbaren, modulierenden und hydrophoben Fluoridenthaltenden Gruppen sind aus EP-0794756-A1 und EP-0794757-A1 (3M) bekannt.

Teilweise sind auch Präparate beschrieben, die Verbindungen enthalten, die sowohl antimikrobielle Aktivität gegenüber bestimmten Bakterienstämmen aufweisen und gleichzeitig deren Adhäsion verringern sollen (Journal of Oral Rehabilitation (1988) 15, 405-411).

Zusammenfassend werden im Stand der Technik folgende Methoden beschrieben:

- Verringerung der Proteinadhäsion durch Oberflächenbehandlung oder durch Modifizierung der Basiskunststoffe,
- Abtötung von Mikroorganismen auf freien Oberflächen durch inkorporierte Biozide.

Die jeweiligen Wirkungen dieser bekannten Verfahren sind jedoch noch nicht zufriedenstellend.

Der vorliegenden Erfindung liegt die Aufgabe zugrunde, Kunststoffe bereitzustellen, die bezüglich ihrer adhäsionsverringernden Eigenschaften bzw. bioziden Wirkungen verbessert sind.

Gelöst wird diese Aufgabe durch speziell modifizierte Kunststoffe, die dadurch gekennzeichnet sind, daß sie adhäsionsverringernde Substanzen in einer solchen Menge aufweisen, daß die Adhäsion von Mikroorganismen auf deren Oberfläche um mindestens 50 % geringer ist als bei nicht-modifizierten Kunststoffen, und daß sie biozide Substanzen in einer solchen Menge inkorporiert haben, daß mindestens 60 % der verbleibenden Mikroorganismen innerhalb von 24 Stunden abgetötet werden.

Besonders im dentalen Bereich liegen dauerhaft feuchte Umgebungsbedingungen vor. Wenn sich auf der Oberfläche von Kunststoffen und Abformmaterialien ein Flüssigkeitsfilm befindet, so ist dieser naturgemäß mit Proteinen bzw. Mikroorganismen durchsetzt, was äußerst unvorteilhaft ist. Durch die Verwendung adhäsionsverringernder Substanzen in Kunststoffen hat man sich nichts davon versprochen, biozide Substanzen in diese zu inkorporieren, da davon ausgegangen wurde, daß Bakterien wegen des adhäsionsverringernden Effektes nur kurzfristig mit den Oberflächen in Berührung kommen, jedenfalls zu kurz, als daß diese biozide Wirkung auftreten könnte. Überaschenderweise ist trotz einer deutlichen Adhäsionsverringerung von Proteinen an den Oberflächen der erfindungsgemäß modifizierten Kunststoffe eine genügend lange Kontaktzeit der Mikroorganismen mit den Oberflächen gegeben, so daß die Mikroorganismen zu einem überwiegenden Anteil abgetötet werden.

Die Erfindung wird nachfolgend detaillierter beschrieben.

Als Kunststoffe können, abhängig vom einzusetzenden Modifikator, alle bekannten Kunststoffarten verwendet werden; exemplarisch aber nicht limitierend seien Polyethylene, Polypropylene, Polyvinylchloride, Polystyrole, Polycarbonate, Cellophane, Celluloseacetate, Polyelofine, fluorierte Kohlenwasserstoffe (Teflon), Polyhydroxyethylmethacrylate (PHEMA) (Hydron), Polymethylmethacrylate (PMMA), Polysiloxane, Polyether und Polyethersilikone erwähnt.

Ein erfindungswesentlicher Schritt besteht darin, Biozide in Kunststoffe einzubringen. Ein anderer erfindungswesentlicher Schritt besteht in der Inkorporierung adhäsionsverringernder Substanzen in die Kunststoffe oder in der Behandlung der Kunststoffoberflächen mit adhäsionsverringernden Substanzen. Die Reihenfolge beider Schritte ist frei wählbar und durch die technischen Verfahrensweisen der Kunststoffherstellung bedingt.

Um biozide Materialien in die Kunststoffe einzubringen, ist beispielsweise die Solvent Casting-Methode (J. M. Schierholz, A. Rump und G. Pulverer, Arzneim. Forsch. (1997) 47, 70ff) geeignet.

Möglich ist es ferner, die ausgehärteten Kunststoffe zu vermahlen, mit den Bioziden zu vermischen und wieder in Form zu pressen, ggf. unter Wärmeeinwirkung. Auch kann das Biozid bereits während des Kunststoff-Spritzvorgangs zugesetzt werden. Ebenso ist die Inkorporierung von Bioziden in dentalen Abformmassen denkbar. Hierbei ist das Biozid einer beliebigen Komponente der nicht-ausgehärteten Masse zuzugeben. Durch den Abbindevorgang wird dann ein Gummi erhalten, der biozide Eigenschaften aufweist. Vorteilhaft ist bei dieser Indikation auch, daß durch die adhäsionsverringernden Eigenschaften Blut- und Speichelreste zunächst vom Abdruckmaterial abfließen und dann vorhandene Mikroorganismen abgetötet werden.

Als Biozide geeignet sind Substanzen, die Silber, Silberionen, Kupfer, Kupferionen, Zink oder Zinkionen freisetzen (MicroFree™ AMP, DuPont), beispielsweise Kupferoxide oder Zinksilikate, aber auch die freien Metalle. Selbstverständlich sind auch alle anderen erhältlichen Biozide einsetzbar. Erwähnenswert sind beispielsweise Ciprofloxacin-HCl (Bayer AG, Leverkusen), Ciprofloxacin-Betain, 10,10-Oxybisphenoxyarsin, 2-n-Octyl-4-isothiazolin-3-on, 2,3,5,6-Tetrachloro-4-(methylsulphonyl)pyridin, N-Trichloromethylthiophthalimid, Chlorhexidin, langkettige Bisphenolester (US 3,427,345; 3M), Natriumfluorid in Kombination mit Dodecylamin oder anderen organischen Aminen, Benzalkoniumchlorid, Cetylpyridiniumchlorid, 4-Chlor-2-(2,4-Dichlorophenoxy)phenol (Triclosan), komplexe oder einfache Fluoride wie $SnF_2$, $KZnF_3$, $ZnSnF_4$, $Zn(SnF_3)_2$, Kalium oder Zirkonhexafluorotitanat, N-Oxide von gesättigten N-haltigen Heterocyclen substituiert durch Quinoloncarboxylsäuren oder Napthyridoncarboxylsäuren (EP-0828715-A1). Die Biozide werden in Mengen von ca. 0,001 bis 20 Gew.-Teilen, vorzugsweise 0,01 bis 10 Gew.-Teilen, pro 100 Gew.-Teile Gesamtmasse eingebracht.

Als adhäsionsverringernde Substanzen und Methoden zur Herstellung adhäsionsverringerter Kunststoffe bzw. Oberflächen eignen sich beispielsweise solche auf Phosphorylcholinbasis oder Phosphorylethanolaminbasis (WO-93/21970, EP-0199790-B1,WO-94/14897, WO-93/01221, EP-0641226-B1, EP-0518959-B1, WO-90/09384, EP-0157469-B1, WO-94/16749, WO-94/16748) oder auch solche auf der Basis von Polyestern aus Einheiten, die von Glycerophosphorylcholin oder Glycerophosphorylethanolamin abgeleitet sind und polyfunktionellen Säuren oder deren Derivaten (EP-0275293-B1). Die adhäsionsverringernden Substanzen werden in Mengen von ca. 0,05 bis 50 Gew.-Teilen, vorzugsweise 0,1 bis 20 Gew.-Teilen, pro 100 Gew.-Teile Gesamtmasse eingebracht oder als Oberflächenbeschichtung aufgebracht.

Im allgemeinen wird die Oberflächenbeschichtung vorgenommen, indem das reaktive Agens in einem kompatiblen Lösungsmittel gelöst, die Oberfläche mit der Lösung behandelt und dann getrocknet wird. Gegebenenfalls muß die Oberfläche funktionalisiert werden; dies kann über bekannte ätzende oder derivatisierende Techniken, wie der Plasmaentladung (siehe dazu "Chemical Reactions of Polymers", Ed. E.M. Fettes (1964), Interscience, London) geschehen. Die diversen Verfahren sind aus den vorher genannten Schriften entnehmbar, deren Offenbarungsgehalt hiermit vollkommen umfaßt sein soll.

Die erfindungsgemäßen Kunststoffe eignen sich zur Herstellung von im dentalen und dentaltechnischen Bereich zu verwendenden Kunststoffen und Kunststoffteilen, beispielsweise Gehäuseteilen von Misch- oder Polymerisationsgeräten, auch Lichtstäben von Lichtpolymerisationsgeräten, Primär- und Sekundärverpackungsmaterialien von Dentalmaterialien, Applikationsinstrumenten für Dentalmaterialien, beispielsweise zum Applizieren von Füllungsmaterialien, sowie für Dentalmaterialien selbst, beispielsweise Abformmaterialien. Allgemein geeignet sind sie für alle Kunststoffteile, die bei der Ausübung des zahnärztlichen oder zahntechnischen Berufes in Verwendung sind.

Nachfolgend werden die Prüfmethoden und Methoden beschrieben, die zum Nachweis der Devitalisierung von Mikroorganismen und der Adhäsionsverringerung von Mikroorganismen heranzuziehen sind.

Prüfmethode 1

Vitalfluoreszenzmethode nach Netuschil (Dtsch, zahnärztl, Z. (1983) 38, 914-917)

Die Anfärbung lebender Zellen wird über Fluoresceindiacetat (FDA) vorgenommen, welches von den Zellen aufgenommen und in Fluorescein umgewandelt wird, das seinerseits die Zellen nicht mehr verlassen kann. Tote Zellen können diesen Reaktion nicht mehr durchführen und müssen daher mit Ethidiumbromid (EB) angefärbt werden. Dieses bindet spezifisch an die DNA des Zellkerns, der nur bei toten Zellen zerstört ist.

Als Reagenzien werden folgende Lösungen verwendet:

- FDA (Sigma F-5502), Stammlösung 5 mg/ml Aceton, jeweils 2 oder 6 µl pro ml physiologischer NaCl-Lösung einzusetzen;
- EB (Sigma E-8751), Stammlösung 1,25 mg/ml physiologischer NaCl-Lösung, jeweils 3 µl/ml physiologischer NaCl-Lösung einzusetzen.

Zum Nachweis des erfindungsgemäßen Effektes werden standardisierte Scheiben hergestellt, die einerseits aus Kunststoffen, die nach den vorher beschriebenen Methoden modifiziert wurden und andererseits aus nicht-modifizierten Kunststoffen bestehen. Diese werden nach dem Herstellungsbeispiel 1 (Punkt 3) für 24 h mit einer Bakterien- und Proteinsuspension in Berührung gebracht.

Ein Vergleich der Plättchen aus erfindungsgemäß modifiziertem und nicht-modifiziertem Kunststoff wird nach dem Anfärben mit FDA und EB unter einem Lichtmikroskop mittels Zählgitter (Leitz Diavert mit Fluoreszenzeinrichtung, Lampe 50 HBO, Blau- und Grünanregung über Filterblock I2 bzw. N2) vorgenommen, indem die Anzahl der vitalen und der devitalen Mikroorganismen durch Abzählen bestimmt wird. Einen prozentualen Wert erhält man durch die

einfach Formel:

[%-Vitalfluoreszenz] = 100 % · [Anzahl vitale Bakterien] / ([Anzahl vitale Bakterien]

+ [Anzahl devitale Bakterien]).

Herstellungsbeispiel 1

Herstellung einer Bakteriensuspension

Zur Überprüfung des erfindungsgemäßen Effektes werden standardisierte Prüfplättchen für 4 h in eine Testkeimlösung getaucht und die Anzahl der adhäsierten Bakterien nach weiteren 24 h Inkubation bestimmt.

1. Anzucht der Testkeime: Streptococcus sanguis, biotype 1, DSM 20068 und Streptococcus mutans, ATCC 25175, DSM 20523 (DSM = Deutsche Sammlung von Mikroorganismen) werden jeweils in einer Flasche mit Caso-Bouillon angezüchtet, indem die beimpfte Bouillon bei 30 - 35 °C inkubiert wird, bis eine starke Trübung vorliegt. Zur Anzucht der Testkeime werden aus dieser Bouillon Platten aus Gaso-Bouillon + 0,5 % Hefeextrakt + 1,5 % Agar mit jeweils 0,5 ml beimpft und 2 - 3 Tage bei 30 - 35 °C bebrütet.

2. Herstellung der Keimsuspension: Mit Hilfe einer Impföse werden die Keime von bewachsenen Platten in Ringerlösung suspendiert, bis eine starke Trübung vorliegt. Die Keimsuspension wird anschließend 10 Minuten im Ultraschallbad behandelt und 1:10 mit synthetischer Speichellösung (Shellis R. P.; Archives of Oral Biology; 23, (1978), 485-489 modifiziert nach Glenister D. A., Salamon K. E., et al.; Microbial Ecology in Health and Disease; 1, (1988), 31-38) verdünnt und homogenisiert. Die fertige Keimsuspension enthält Streptococcus mutans ca. $10^7$ -$10^8$ KBE / ml und Streptococcus sanguis ca. $10^8$ - $10^7$ KBE / ml.

3. Testansatz: Die Prüfplättchen werden in 70 %-igem Ethanol desinfiziert. Nach dem Trocknen werden alle Plättchen mit Hilfe einer sterilen Pinzette zusammen in eine sterile 100 ml-Flasche überführt und mit 10 ml der Keimsuspension versetzt, wobei die Suspension jedesmal vor Entnahme durch Schütteln gut homogenisiert werden muß. Anschließend werden die Proben für 4 Stunden bei 37 °C und 100 rpm im Schüttelwasserbad inkubiert.

Herstellungsbeispiel 2

Herstellung eines antibiotikahaltigen Polyurethans (Solvent Casting)

Polyurethan "Walopur" (Wolff, Walsrode) wird 24 h in einer Wasser/Ethanol-Mischung (1:1) bei 82°C unter Rückfluß extrahiert und anschließend in Dimethylformamid (102°C, Rückfluß) gelöst. Ciprofloxacin-Betain wird in dieser Lösung mit einer Konzentration von 750 ppm gelöst und das Lösungsmittel bei 50°C und 400 mbar über 24 h abgezogen, der Rest zu Plättchen mit 1,5 cm Durchmesser und 0,5 mm Dicke geformt, diese mit deionisiertem Wasser gewaschen und sorgfältig getrocknet.

Herstellungsbeispiel 3

Herstellung eines antibiotikahaltigen plastifiziertem Polyvinylchorids

Zur Herstellung von plastifiziertem Polyvinylchlorid (pPVC) werden folgende Bestandteile verwendet:

| Komponente | Teile auf 100 Harzteile |
|---|---|
| **571/102 Corvic (PVC-Harz)** | 100 |
| **Dioctylphthalat (Plastifizierer)** | 25 |
| **Dioctyladipat (Plastifizierer)** | 25 |
| **Lankromark LZ 935 (AKCROS) (Calcium/Zink Stabilisierer)** | 2 |
| **Lankroflex Ed 6 (AKCROS) (Epoxidierter Oleatester)** | 3 |

(fortgesetzt)

| Komponente | Teile auf 100 Harzteile |
|---|---|
| **Stearinsäure** | 0,2 |
| **Calciumstearat** | 0,5 |

Das Biozid 10,10-Oxybisphenoxyarsin wurde zu 750 ppm gleichzeitig mit den Plastifizierern in die pPVC-Formulierung eingebracht. Die Mischung wurde in einer Zweiwalzenmühle bei 160°C 1,5 bis 2 min. gemischt und auf einer hydraulischen 50t-Parallelpresse (Moore) bei 160°C in 30 $cm^2$ große 0,5 mm dicke Schichten laminiert.
Aus diesen Schichten wurden 1,5 cm große Plättchen gestanzt.

Herstellungsbeispiel 4

Oberflächenbeschichtung zur Adhäsionsverringerung

Die Plättchen aus den Herstellungsbeispielen 2 oder 3 werden in eine ethanolische Lösung von präpolymerisiertem Diacetylphosphatidylcholin, hergestellt nach der Vorschrift A3 der WO-93/21970, getaucht, herausgenommen, von Überschüssen durch Abtropfen befreit und bei Raumtemperatur getrocknet.
Der erfindungsgemäße Effekt wird aus nachfolgender Übersicht deutlich. Es wurden hierbei jeweils drei Plättchen aus erfindungsgemäßem Kunststoff und drei Plättchen aus nicht-modifiziertem Kunststoff in der Bakterien- und Proteinlösung aus Herstellungsbeispiel 1 für 24 Stunden belassen, danach unter dem Lichtmikroskop analog Methode 1 die Gesamtzahl der Bakterien auf erfindungsgemäßem Kunststoff sowie die Zahl der Bakterien auf nicht-modifiziertem Kunststoff ermittelt, daraus die prozentuale Adhäsion bestimmt und anschließend die Zahl der toten und der lebenden Bakterien auf dem erfindungsgemäßen Kunststoff gezählt und die Vitalfluoreszenz ermittelt.
Prozentzuale Werte erhält man durch die einfachen Formeln:

[%-Adhäsion] = 100% · [Anzahl Bakterien auf erfindungsgemäßem Kunststoff] /

[Anzahl Bakterien auf nicht-modifiziertem Kunststoff]

[%-Vitalfluoreszenz] = 100% · [Anzahl vitale Bakterien] / ([Anzahl vitale Bakterien]

+ [Anzahl devitale Bakterien])

Die Proben 1 bis 3 repräsentieren die Durchschnittswerte jeweils dreier Kunststoffplättchen nach den Herstellungsbeispielen 2 und 4, die Proben 4 bis 6 solche nach den Herstellungsbeispielen 3 und 4.

| Probe | %-Adhäsion | %-Vitalfluoreszenz |
|---|---|---|
| 1 | 49 | 5 |
| 2 | 30 | 3,7 |
| 3 | 38 | 1,2 |
| 4 | 33 | 0,05 |
| 5 | 23 | 1 |
| 6 | 19 | 0,5 |

**Patentansprüche**

1. Modifizierte Kunststoffe, **dadurch gekennzeichnet, daß** sie adhäsionsverringernde Substanzen in einer solchen Menge aufweisen, daß die Adhäsion von Mikroorganismen auf deren Oberfläche um mindestens 50 % geringer ist als bei nicht-modifizierten Kunststoffen, und daß sie biozide Substanzen in einer solchen Menge inkorporiert haben, daß mindestens 60% der verbleibenden Mikroorganismen innerhalb von 24 Stunden abgetötet werden.

2. Modifizierte Kunststoffe nach Anspruch 1, **dadurch gekennzeichnet, daß** sie adhäsionsverringernde Substanzen inkorporiert haben.

3. Modifizierte Kunststoffe nach Anspruch 1, **dadurch gekennzeichnet, daß** deren Oberflächen durch adhäsionsverringernde Substanzen modifiziert sind.

4. Modifizierte Kunststoffe nach Anspruch 1, **dadurch gekennzeichnet, daß** sie als biozide Substanzen Silber, Silberionen freisetzende Substanzen, Kupfer, Kupferionen freisetzende Substanzen, Zink oder Zinkionen freisetzende Substanzen inkorporiert haben.

5. Verfahren zur Herstellung modifizierter Kunststoffe nach einem der Ansprüche 1 bis 4 , **dadurch gekennzeichnet, daß** die adhäsionsverringernden und bioziden Substanzen in die Kunststoffe eingebracht werden.

6. Verfahren zur Herstellung modifizierter Kunststoffe nach einem der Ansprüche 1 bis 4, **dadurch gekennzeichnet, daß** die adhäsionsverringernden Substanzen auf die Oberfläche der Kunststoffe aufgebracht und die bioziden Substanzen in die Kunststoffe inkorporiert werden.

7. Verwendung der modifizierten Kunststoffe nach einem der Ansprüche 1 bis 4 zur Herstellung von Formkörpern für Gerätschaften, Gerätekomponenten, primären und sekundären Verpackungsmaterialien, Applikationsinstrumenten sowie Abformmaterialien für den dentalen und dentaltechnischen Bereich.

## Claims

1. Modified plastics, **characterized in that** they contain adhesion-reducing substances in such an amount that the adhesion of microorganisms to their surface is lower by at least 50% than in the case of non-modified plastics, and **in that** they have incorporated biocidal substances in such an amount that at least 60% of the remaining microorganisms are destroyed within 24 hours.

2. Modified plastics according to Claim 1, **characterized in that** they have incorporated adhesion-reducing substances.

3. Modified plastics according to Claim 1, **characterized in that** their surfaces are modified by adhesion-reducing substances.

4. Modified plastics according to Claim 1, **characterized in that**, as biocidal substances, they have incorporated silver, substances liberating silver ions, copper, substances liberating copper ions, zinc or substances liberating zinc ions.

5. Process for the preparation of modified plastics according to one of Claims 1 to 4, **characterized in that** the adhesion-reducing and biocidal substances are introduced into the plastics.

6. Process for the preparation of modified plastics according to one of Claims 1 to 4, **characterized in that** the adhesion-reducing substances are applied to the surface of the plastics and the biocidal substances are incorporated into the plastics.

7. Use of the modified plastics according to one of Claims 1 to 4 for the production of moulded articles for implements, apparatus components, primary and secondary packaging materials, application instruments and casting materials for the dental and prosthodontic field.

## Revendications

1. Matières synthétiques modifiées, **caractérisées en ce qu'**elles présentent des substances qui diminuent l'adhérence en quantités telles que l'adhérence de microorganismes sur leur surface soit d'au moins 50% inférieure à celle sur des matières synthétiques non modifiées, et **en ce qu'**on y a incorporé des substances biocides en quantités telles qu'au moins 60% des microorganismes résiduels soient tués en 24 heures.

2. Matières synthétiques modifiées selon la revendication 1, **caractérisées en ce qu'**on y a incorporé des substances qui diminuent l'adhérence.

3. Matières synthétiques modifiées selon la revendication 1, **caractérisées en ce que** leur surface a été modifiée par des substances qui diminuent l'adhérence.

4. Matières synthétiques modifiées selon la revendication 1, **caractérisées en ce que** l'on y a incorporé comme substances biocides de l'argent, des substances qui libèrent des ions argent, du cuivre, des substances qui libèrent des ions cuivre, du zinc ou des substances qui libèrent des ions zinc.

5. Procédé de préparation de matières synthétiques modifiées selon l'une quelconque des revendications 1 à 4, **caractérisé en ce que** les substances qui diminuent l'adhérence et les substances biocides sont incorporées dans les matières synthétiques.

6. Procédé de préparation de matières synthétiques modifiées selon l'une quelconque des revendications 1 à 4, **caractérisé en ce que** les substances qui diminuent l'adhérence sont appliquées sur la surface des matières synthétiques et **en ce que** les substances biocides sont incorporées dans les matières synthétiques.

7. Utilisation des substances synthétiques modifiées selon l'une quelconque des revendications 1 à 4 pour la préparation de corps façonnés pour des manches d'appareils, des composants d'appareils, des matériaux d'emballage primaire et secondaire, des instruments d'application ainsi que des matériaux de moulage dans le domaine dentaire et le domaine de la technique dentaire.